# EUROPEAN PATENT APPLICATION

(11) **EP 1 529 514 A1**
(43) Date of publication of application: **11.05.2005**
(21) Application number: 04292634.5
(22) Date of filing: 05.11.2004
(51) Int. Cl.: A61K 7/02

(54) **Make-up composition comprising a particulate material enclosing colored inorganic pigments and inorganic pigments**

(30) Priority: 07.11.2003 JP 2003378103
(71) Applicant: L'OREAL, 75008 Paris (FR)
(72) Inventor: Dumousseaux, Christophe, c/o Nihon L'Oreal K.K., Kawasaki-shi 213-0012 Kanagawa (JP)
(74) Representative: Le Coupanec, Pascale

(57) **Abstract**

The present invention relates to a make-up cosmetic composition comprising 0.5 to 25%, preferably 1 to 20%, more preferably 1 to 15% by weight of particulate material with volume average particle size of 1 to 20 µm and enclosing colored inorganic pigments and 0.2 to 8%, preferably 0.5 to 7, more preferably 1 to 6 by weight of white and/or colored inorganic pigments. This make-up cosmetic composition can provide sufficient hiding power and transparency as well as natural appearance after application on skin.

## Description

This invention relates to make-up composition containing 0.5% to 25% by weight particulate material with a volume average particle size of 1 to 20 µm enclosing colored inorganic pigments and 0.2% to 8% by weight of white and colored inorganic pigments. This cosmetic make-up composition imparts good coverage together with an excellent feeling of transparency on human skin.

One of the main purposes of make-up foundation is to provide smooth and even looking skin tone. This effect is traditionally obtained by adding in the make-up cosmetic different kind of powders. For example, conventional inorganic pigments, such as titanium oxide and iron oxides, have been used in cosmetic make-up composition to cover defects on skin such as blots or freckles and to finally try to make an even looking. However the use of a high level of these pigments tends to make the appearance very unnatural, also called mask-like appearance. At the same time, an accumulation of pigments is often observed in fine lines and wrinkles, and instead of hiding these defects, the make-up composition tends to highlight them.

In order to solve this problem, transparent fillers of different shape have been used in combination with inorganic pigments. These fillers combine a good transparency and a strong diffusion of light. They are traditionally helping to obtain a good soft focus effect (R. Emmert, Cosmetic&Toiletries, vol.111, no.7, p.57 (1996)). However, when these fillers are used in combination with a high level of inorganic pigment, the transparency of the formulation tends to be very low and the soft focus effect is completely lost. On the contrary, if no pigments or a very low level of pigments are used the covering power of the make-up product is not sufficient to reduce the appearance of skin discolorations.

Attempts have been made to use composite particles that combine pigments and fillers. Pigments can be enclosed and dispersed inside different kind of fillers, or pigments can be applied on the surface of fillers. However the use of this type of pigments alone will not give suitable covering power or the use in an inappropriate ratio of traditional pigments will cancel the benefits of these composite particles.]

There is therefore a big need for a make-up composition that can be at the same time very transparent and appear natural after application on skin, together with a high covering power after application on skin.

We have discovered that by associating in a right proportion particulate material enclosing colored inorganic pigments and white and/or colored inorganic pigments it is possible to obtain make-up composition with satisfactory hiding effect together with a high feeling of transparency.

According to a first aspect, the invention is directed to a make-up cosmetic composition comprising 0.5 to 25% by weight of particulate material with volume average particle size of 1 to 20 µm and enclosing colored inorganic pigments and 0.2 to 8% by weight of white and/or colored inorganic pigments.

In particularly, the composition may be a foundation.

According to a second aspect, the invention is directed to a cosmetic process of make-up of the skin comprising at least a step of applying on the skin a composition according to the invention.

According to a third aspect, the invention is directed to the use of a combination of 0.5 to 25% by weight of particulate material with volume average particle size of 1 to 20 µm and enclosing colored inorganic pigments and 0.2 to 8% by weight of white and/or colored inorganic pigments in a make-up cosmetic composition for the skin in order to obtain make-up compositions with hiding effect together with feeling of transparency.

The invention is also directed to the use of a make-up composition according to the invention for obtaining a make-up of the skin having hiding effect together with feeling of transparency.

The make-up composition of the invention contains 0.5 to 25 wt%, preferably 1 to 20 wt%, more preferably 1 to 15 wt% of particulate material enclosing colored inorganic pigments and 0.2 to 8 wt%, preferably 0.5 to 7 wt%, more preferably 1 to 6 wt% of white and colored inorganic pigments.

The particulate material have a volume average particle size between 1 and 20 µm. For smaller particle size it would be very difficult to introduce and disperse homogeneously the pigments inside the material. For larger particle size, sensorial properties during application and optical properties after application will be decreased. Larger colored particulate size will have a high forward scattering and therefore bring almost no covering power in the formulation. The volume average particle size can be measured with a powder laser granulometer, either in air or in ethanol medium. Commercial instruments are available from Malvern Instrument, Horiba Ltd or Coulter companies.

By enclosing colored inorganic pigment it is meant that the pigment are located inside the particulate material. Therefore the composite particulate of the present invention does not refer to particles which are covered or coated by one layer or multilayer of pigments at their surface. In the composite particulate material of the present invention inorganic pigments have to be well and homogeneously dispersed inside the particulate material. The appropriate choice of material and the percentage of pigments inside enable to have a good compromise of optical properties. Process to obtain this kind of composite material is for example well described in JP-B-2591946 (Sumitomo Chemical), JP-B2861806 (NSG), JP-A-H08-239310 (NSG), JP-A-H06-47273 (Suzuki Oil and Fat) in the case of inorganic based composite material, and in JP-A-2001-354776 (Shinetsu Chemical) in the case of silicone elastomer based particles. An advantage of this type of composite material is the absence of interaction between the pigments and water or oil. There will be therefore no change of color after application of the make-up composition, no dulling effect with time when sweat or sebum start to wet the composition.

By colored inorganic pigments it is meant metal oxide pigments traditionally used to match the skin color in cosmetic. The color of these pigments could be red, yellow, blue, green, violet, brown or black. Typical example includes iron oxide, ultramarine blue, Prussian blue, ferric blue, manganese violet, and chromium oxide. It also includes mixed or complex pigments comprising mixture of metal oxides like mixed titanium-iron oxides. These pigments have a primary particle size from 0.1 to 1.5 µm. Smaller and higher particle size will not be adequate in terms of hiding power.

The base material can be organic or inorganic. Organic material include cellulose-based polymer, silicon resin, silicon elastomer, acrylic polymer, polyurethane based polymer, polyamide, polyethylene, polystyrene. Inorganic material includes glass, silicon dioxide or silicium-based composite oxide.

According to a specific embodiment, the particulate material enclosing colored pigments consists of cellulose based polymer or silica based material.

There is no limitation in terms of shape of the particulate material, including sphere, flake, rod or needle. The particulate material can be porous or nonporous.

The particulate material should have a refractive index below 1.8, preferably below 1.6, in order to enhance the transparency after application on skin. In fact the transparency of these material, when wetted by the oil present in the formulation, tends to be high.

Percentage of inorganic pigments inside the particulate material should be ranging from 3 to 60wt%. If the percentage is lower, the particulate material will not give enough hiding power. If the percentage is higher, it tends to be difficult to satisfactorily synthesize the material. Particulate material may enclose one type of colored inorganic pigment, a mixture of colored inorganic pigment or a mixture of white and colored inorganic pigments.

Examples of commercially available particulate material enclosing colored inorganic pigments include products based on polymethylmethacrylate available from Ganz Chemical (Ganzpearl®), products based on cellulose available from Daito (Cellulobeads® ), products based on porous silica available from Miyoshi (PC Ball®).

The inorganic pigments used in combination with the particulate material enclosing inorganic pigments may be white pigments or colored pigments or a mixture thereof.
By white inorganic pigment it is meant white metal oxide pigments traditionally used in cosmetic application, which may be surface-treated. Typical examples are titanium oxide, zinc oxide, cerium oxide or zirconium oxide. These pigments have a primary particle size from 0.1 to 1.5 µm. Smaller and higher particle size will not be adequate in terms of hiding power.

The colored inorganic pigments are as described above.

The pigments could be surface-treated with a hydrophobic agent in order to improve their dispersibility in the fatty phase. The hydrophobic treatment agent may be chosen from silicones such as methicones, dimethicones, perfluoroalkylsilanes; fatty acids such as stearic acid; metal soaps such as aluminum dimyristate, the aluminum salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups, amino acids; N-acylated amino acids or their salts; lecithin, isopropyl triisostearyl titanate, and mixtures thereof.

The N-acylated amino acids may comprise an acyl group having from 8 to 22 carbon atoms, such as for example a 2-ethylhexanoyl, caproyl, lauroyl, myristoyl, palmitoyl, stearoyl and cocoyl group. The salts of these compounds may be the aluminium, magnesium, calcium, zirconium, zinc, sodium and potassium salts. The amino acid may be, for example, lysine, glutamic acid and alanine.
The term alkyl mentioned in the compounds cited above denotes in particular an alkyl group having from 1 to 30 carbon atoms, preferably having from 5 to 16 carbon atoms.

The composition may also comprise at least one dyestuff, for instance pulverulent compounds and/or liposoluble dyes, for example in a proportion of from 0.01% to 50% relative to the total weight of the composition. The pulverulent compounds may be chosen from the pigments and/or nacres usually used in cosmetic compositions. Advantageously, the pulverulent compounds represent from 0.1% to 25% of the total weight of the composition, and better still from 1 % to 20%.

Among the organic pigments that may be mentioned are pigments of D & C type and lakes based on cochineal carmine or on barium, strontium, calcium or aluminum. The nacreous pigments may be chosen from white nacreous pigments such as mica coated with titanium or with bismuth oxychloride, colored nacreous pigments such as titanium mica with iron oxides, titanium mica with, in particular, ferric blue or with chromium oxide, titanium mica with an organic pigment of the abovementioned type, and also nacreous pigments based on bismuth oxychloride.

The composition may also comprise fillers, which may be chosen from those that are well known to those skilled in the art and which are commonly used in cosmetic compositions. The fillers may be mineral or organic, and lamellar or spherical. Mention may be made of talc, mica, silica, kaolin, Nylon powder (Orgasol® from Atochem), poly-β-alanine powder, polyethylene powder, Teflon® lauroyllysine, starch, boron nitride, tetrafluoroethylene polymer powders, hollow microspheres such as Expancel® (Nobel Industrie), Polytrap® (Dow Corning), silicone resin microbeads (for example Tospearls® from Toshiba), precipitated calcium carbonate, magnesium carbonate, magnesium hydrocarbonate, hydroxyapatite, hollow silica microspheres (Silica Beads® from Maprecos), glass or ceramic microcapsules, metal soaps derived from organic carboxylic acids containing from 8 to 22 and preferably from 12 to 18 carbon atoms, for example zinc stearate, magnesium stearate, lithium stearate, zinc laurate or magnesium myristate.

The composition may also contain any additive usually used in such compositions, such as thickeners, preserving agents, fragrances, sunscreens, free-radical scavengers, waxes, moisturizers, vitamins, proteins, sequestrants, ceramides, acidifying or basifying agents, or emollients.

The composition can contain volatile oil. The expression ''volatile oil" means any non-aqueous medium that is capable of evaporating from the skin in less than one hour. This volatile phase in particular comprises oils with a vapour pressure, at room temperature and atmospheric pressure, ranging from 10⁻² to 300 mmHg (1,33 Pa to 40 000 Pa).

These volatile oils can be hydrocarbon-based oils or silicone oils optionally comprising alkyl or alkoxy groups at the end of the silicone chain or pendent on the chain.

As volatile silicone oils which can be used in the invention, mention may be made of linear or cyclic silicones containing from 2 to 7 silicon atoms, these silicones optionally comprising alkyl or alkoxy groups containing from 1 to 10 carbon atoms. Mention may be made in particular of octamethylcyclotetrasiloxane, decamethylcyclopentasiloxane, hexadecamethylcyclohexasiloxane, heptamethylhexyltrisiloxane and heptamethyloctyltrisiloxane.

Volatile hydrocarbon-based oils that may be mentioned are C₈-C₁₆ isoparaffins such as isooctane, isododecane, isodecane, heptane, isohexadecane and/or mixtures thereof.

The composition can also comprise at least one non-volatile oil chosen in particular from non-volatile hydrocarbon-based and/or silicone and/or fluoro oils.

Non-volatile hydrocarbon-based oils which may be mentioned in particular are:
- hydrocarbon-based plant oils such as triglycerides consisting of fatty acid esters and of glycerol in which the fatty acids may have varied chain lengths from C₄ to C₂₄, these chains possibly being linear or branched, and saturated or unsaturated; these oils are, in particular, wheat germ oil, sunflower oil, grape seed oil, sesame oil, corn oil, apricot oil, castor oil, karite butter, avocado oil, olive oil, soybean oil, sweet almond oil, palm oil, rape seed oil, cotton oil, hazelnut oil, macadamia oil, jojoba oil, alfalfa oil, poppy oil, pumpkin oil, marrow oil, blackcurrant seed oil, evening primrose oil, millet oil, barley oil, quinoa oil, rye oil, safflower oil, candlenut oil, passion flower oil and musk rose oil; or alternatively caprylic/capric acid triglycerides such as those sold by Stearineries Dubois or those sold under the names Miglyol 810, 812 and 818 by Dynamit Nobel;
- synthetic ethers containing from 10 to 40 carbon atoms;
- linear or branched hydrocarbons of mineral or synthetic origin, such as petroleum jelly, polydecenes, hydrogenated polyisobutene such as parleam, and squalane, and mixtures thereof;
- synthetic esters such as oils of formula R₁COOR₂ in which R₁ represents a linear or branched fatty acid residue containing from 1 to 40 carbon atoms and R₂ represents an in particular branched hydrocarbon-based chain containing from 1 to 40 carbon atoms, on condition that R₅ + R₆ is larger than 10, such as, for example, purcellin oil (cetostearyl octanoate), isopropyl myristate, isopropyl palmitate, C₁₂-C₁₅ alkyl benzoate, hexyl laurate, diisopropyl adipate, isononyl isononanoate, 2-ethylhexyl palmitate, isostearyl isostearate, alkyl or polyalkyl octanoates, decanoates or ricinoleates such as propylene glycol dioctanoate; hydroxylated esters such as isostearyl lactate and diisostearyl malate; and pentaerythritol esters;
- fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;
- higher fatty acids such as oleic acid, linoleic acid or linolenic acid;
and mixtures thereof.

The non-volatile silicone oils which may be used in the composition according to the invention may be non-volatile polydimethylsiloxanes (PDMSs), polydimethylsiloxanes comprising alkyl or alkoxy groups, that are pendent and/or at the end of a silicone chain, the groups each containing from 2 to 24 carbon atoms, phenylsilicones, for instance phenyltrimethicones, phenyldimethicones, phenyltrimethylsiloxydiphenylsiloxanes, diphenyldimethicones, diphenylmethyldiphenyltrisiloxanes and 2-phenylethyl trimethylsiloxysilicates.
The fluoro oils which may be used in the invention are, in particular, fluorosilicone oils, fluoropolyethers or fluorosilicones, as described in document EP-A-847 752.

The composition could contain an aqueous phase containing water. The water may be a floral water such as cornflower water and/or a mineral water such as VITTEL water, LUCAS water or LA ROCHE POSAY water and/or a thermal water.

The aqueous phase may also comprise solvents other than water, such as for example primary alcohols such as ethanol and isopropanol, glycols such as propylene glycol, butylene glycol, dipropylene glycol, diethylene glycol, glycol ethers such as (C₁-C₄)alkyl ether of mono-, di- or tripropylene glycol, mono-, di- or triethylene glycol, and mixtures thereof.
The aqueous phase may comprise, in addition, stabilizing agents, for example sodium chloride, magnesium dichloride and magnesium sulphate.

Following non-limiting examples and comparative examples are given to illustrate the invention. All compositions are written with percentages by weight.

### (Example 1 and Comparative Examples 1 to 3)

The liquid foundations having the following compositions were prepared.

**[Table 1]**

| | Ex. 1 | Comp. Ex. 1 | Comp. Ex.2 | Comp. Ex. 3 |
|---|---|---|---|---|
| Phase I | | | | |
| Dimethicone copolyol | 5 | 5 | 5 | 5 |
| Dimethicone | 4 | 4 | 4 | 4 |
| Cyclomethicone | 14.5 | 14.5 | 9.5 | 17.5 |
| Isododecane | 10.5 | 10.5 | 10.5 | 10.5 |
| Bentone gel | 10 | 10 | 10 | 10 |
| Plastic Powder D400¹ | 4 | 8 | 4 | 4 |
| Cellulobeads D-5 (W)² | 6.7 | 6.7 | 6.7 | - |
| Cellulobeads D-5 (Y)² | 1.0 | 1.0 | 1.0 | - |
| Cellulobeads D-5 (R) ² | 0.3 | 0.3 | 0.3 | - |
| Aminoacid treated Titanium oxide³ | 3.3 | - | 7.4 | 7.4 |
| Aminoacid treated Iron oxide³ | 0.7 | - | 1.6 | 1.6 |

| Phase II | | | | |
|---|---|---|---|---|
| Water | 31.2 | 31.2 | 31.2 | 31.2 |
| Butylene glycol | 8 | 8 | 8 | 8 |
| Magnesium sulfate | 0.8 | 0.8 | 0.8 | 0.8 |

| | | | | |
|---|---|---|---|---|
| ¹ manufactured by Toshiki Pigment | | | | |
| ² Cellulobeads are spherical cellulose beads manufactured by Daito Kasei and are enclosing 33% of titanium oxide (W) or yellow iron oxide (Y) or red iron oxide (R) pigments respectively. Volume average particle diameter is 8.3 µm for Cellulobeads D-5 (Y) and 9.9 µm for Cellulobeads D-5 (R). | | | | |
| ³NAI series of titanium oxide and iron oxide available from Miyoshi | | | | |

Amino acid treated titanium oxide and iron oxide were premixed with a part of cyclomethicone on a 3 roll mill and added to the rest of Phase I. Phase II was mixed separately, then added to phase I using a conventional homogeneizer.
For these examples of composition hiding power and transparency were measured.

### Measurement of Hiding Power:

Samples were applied on Erichsen hiding power charts (Type 24/5) with a 30 µm thickness applicator and let dry 2 hours at room temperature. The color difference ΔE between the black and the white side of the chart was then measured with a Minolta CR-300 colorimeter in a reflectance mode. The color difference is obtained using Hunter's color difference formula ΔE=[(ΔL)²+(Δa)²+(Δb)²]^{0,5} . The reported value is an average of 9 measurements.

### Measurement of Transparency:

Mean value between 400 and 700 nm of the total transmittance was measured using an integrating sphere with a JASCO (Nihon Bunkou) V-550 spectrophotometer. The reported value is an average of 6 measurements. All measurements were made using a SPF quartz cell (20 µm thickness) on a film after drying 10 minutes at 37 °C. The results are reported in Table 2.

**[Table 2]**

| | Ex. 1 | Comp. Ex. 1 | Comp. Ex.2 | Comp. Ex. 3 |
|---|---|---|---|---|
| Hiding Power (ΔE) | 27 | 38 | 21 | 25 |
| Transparency (%) | 39 | 73 | 22 | 27 |

It is apparent from Table 2 that the composition of the present invention shows a very good compromise between hiding power and transparency. Furthermore this composition has a good retention of color with time after application on skin.

### (Example 2)

A powder foundation having the following composition was prepared.

| | Ex. 2 |
|---|---|
| Talc | 37.85 |
| Sericite | 31.5 |
| Mica | 4.5 |
| Titanium oxide | 4.9 |
| Iron oxide | 1.98 |
| Zinc stearate | 0.9 |
| Liquid paraffin | 3.4 |
| Phenyl Trimethicone | 4.5 |
| Parabens | 0.27 |
| Cellulobeads D-5 (W) | 8.4 |
| Cellulobeads D-5 (Y) | 1.2 |
| Cellulobeads D-5 (R) | 0.4 |

Formulation of example 2 has a good spreadability to skin, gives a natural finish with a good covering effect, makes fine lines and wrinkles hard to be seen and has a good retention of color.

## Claims

1. Make-up cosmetic composition comprising 0.5 to 25% by weight of particulate material with volume average particle size of 1 to 20 µm and enclosing colored inorganic pigments and 0.2 to 8% by weight of white and/or colored inorganic pigments.

2. Composition according to Claim 1, **characterized in that** the composition contains 1 to 20%, preferably 1 to 15% by weight of particulate material enclosing colored inorganic pigments and 0.5 to 7%, preferably 1 to 6% by weight of white and/or colored inorganic pigments.

3. Composition according to Claim 1 or 2, **characterized in that** the particulate material enclosing colored pigments consists of cellulose based polymer, silicone resin, silicone elastomer, acrylic polymer, polyurethane based polymer, polyamide, polyethylene, polystyrene, or silica based materials including glass, silicon dioxide or silicon based composite oxide.

4. Composition according to Claim 1 to 3, **characterized in that** the particulate material enclosing colored pigments consists of cellulose based polymer or silica based material.

5. Composition according to Claim 1 to 4, **characterized in that** the particulate material enclosing colored pigments has a refractive index of less than 1.8.

6. Composition according to any one of Claims 1 to 5, **characterized in that** the colored inorganic pigments enclosed in the particulate material are selected from iron oxide, ultramarine blue, Prussian blue, ferric blue, manganese violet, chromium oxide, and mixed or complex pigments comprising mixture of metal oxide including mixed titanium-iron oxide.

7. Composition according to any one of Claims 1 to 6, **characterized in that** the colored inorganic pigments enclosed in the particulate material have a primary particle size of 0.1 to 1.5 µm.

8. Composition according to any one of Claims 1 to 7, **characterized in that** the colored inorganic pigments enclosed in the particulate material represent 3 to 60% by weight.

9. Composition according to any one of Claims 1 to 8, **characterized in that** the composition is a foundation.

10. Cosmetic process of make-up of the skin comprising a step of applying the composition according to any one of Claims 1 to 10 to the skin.

11. Use of a combination of 0.5 to 25% by weight of particulate material with volume average particle size of 1 to 20 µm and enclosing colored inorganic pigments and 0.2 to 8% by weight of white and/or colored inorganic pigments in a make-up cosmetic composition for the skin in order to obtain make-up composition s with hiding effect together with feeling of transparency.

12. Use according to claim 11, **characterized in that** the particulate material enclosing colored inorganic pigment is as defined in anyone of claim 1 to 5 and the colored inorganic pigment as defined in anyone of claim 6 to 8.

13. Use of make-up composition according to any one of claims 1 to 10 in order to obtain a make-up of the skin having hiding effect together with feeling of transparency.
